(19) Europäisches Patentamt
European Patent Office
Office européen des brevets

(11) EP 2 039 338 A1

(12) **EUROPEAN PATENT APPLICATION**

(43) Date of publication:
**25.03.2009 Bulletin 2009/13**

(21) Application number: **07291118.3**

(22) Date of filing: **20.09.2007**

(51) Int Cl.:
***A61K 8/04*** *(2006.01)* ***A61K 8/81*** *(2006.01)*
***A61Q 5/02*** *(2006.01)* ***A61Q 19/10*** *(2006.01)*

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Rhodia Opérations**
**93306 Aubervilliers (FR)**

(72) Inventors:
- **Anthony, Olivier**
  **95630 Meriel (FR)**
- **Bzducha, Wojciech**
  **92400 Courbevoie (FR)**

(74) Representative: **Boittiaux, Vincent et al**
**Rhodia Services Dir. Pro. Ind.**
**40 Rue de la Haie Coq.**
**93300 Aubervilliers (FR)**

(54) **Highly foaming composition**

(57) The invention relates to high foaming composition comprising a surfactant, and an agent enhancing foaming properties. The agent is a random copolymer comprising from 0,1 to 20% molar of cationic units C deriving from a cationic monomer C, comprising a quaternary ammonium group, and from 80 to 99,9% molar of neutral hydrophilic units Nphilic deriving from a neutral monomer Nphilic.

EP 2 039 338 A1

## Description

Background of the invention

The invention relates to high foaming compositions comprising a surfactant, and an agent enhancing foaming properties.

Various foaming compositions are used in the industry, for example in detergents. Such compositions typically comprise a surfactant that provides foam. The surfactant can also provide detergency. For some compositions, foam is useful for suspending and/or removing some matter. For example in cosmetics, more particularly in rinse off cosmetic compositions such as shampoos and/or shower gels, foam can participate in suspending soil and/or dirt (for example sebum and/or dust) to be removed from hair and/or skin.

In hand-dish washing compositions, foam can participate in suspending soil removed from the table-ware being cleaned, and in avoiding re-soiling onto the cleaned table-ware. In hand-laundry compositions foam can participate in suspending soil removed from the textile articles being cleaned, and in avoiding re-soiling onto the cleaned textile articles. Moreover, in hand cleaning procedure, the presence of foam is a signal for the user of a good cleaning capability. If foam is not present anymore, users tend to add some more cleaning composition, which is sometimes not necessary, and which can thus impact the environment (or which can provide a perception of an impact on the environment). There is a need for compositions that provide improved foam properties such as more foam and/or maintenance of foam, including for example maintenance over addition of soil.

Laundry procedures might depend on the individual performing the laundry, and on the equipment. In some developing countries, some people re-use foamed laundry washes to clean several loads of textile articles (for example several families clean their loads of textile articles, subsequently, using the same equipment and the same wash). People can for example use a semi-automatic equipment to do so. Semi-automatic laundry equipments comprise an open-air motorized tank that rotates or oscillates comprising a foamed laundry wash. One typically dilutes a laundry composition with water into the tank, to obtain a wash medium, foam the medium by motion of the tank and wash load of textile articles into the foam wash medium. Once the load is cleaned, one removes the load from the tank, and another load is then washed. The foam can participate in suspending soil removed from the textile articles being cleaned, and in avoiding re-soiling onto the cleaned textile articles. It is important and economically efficient and environmentally efficient, that foam persists over several washes. However presence of soil, especially greasy soil, and more particularly addition of soil with the next laundry load, tends to destroy the foam. There is a need for compositions that provide improved foam maintenance, more particularly over addition of soil, typically at addition of another load of textile articles.

As a counterexample, one sometimes uses low foaming detergent compositions. Detergents for automatic laundry for example do not provide foam, otherwise it would deteriorate the equipment. The same applies to detergents for automatic dishwashing. To avoid foaming these composition usually comprise an anti-foaming agent, for example a polyorganosiloxane-based anti-foaming agent.

Other uses of high foaming compositions include fire-extinguishing, tunneling, excavation of materials in civil engineering, and preparation of light-weight materials.

There is a need in high foaming compositions, comprising a surfactant, with enhanced foam, for example improved foam maintenance. There is more particularly a need for compositions with improved foam maintenance in the presence of soil or external materials (such as excavating materials or materials used to make a lightweight material), more particularly upon addition of soil.

Summary of the invention

The invention addresses at least one of the needs mentioned above by providing a highly foaming composition comprising:

a) at least one surfactant, and
b) a random copolymer comprising:

- from 0,1 to 20% molar, preferably from 1 to 10%, of cationic units C deriving from a cationic monomer C, comprising a quarternary ammonium group,
- from 80 to 99,9% molar, preferably from 90 to 99%, of neutral hydrophilic units $N_{philic}$ deriving from a neutral hydrophilic monomer $N_{philic}$, and
- optionally further units F different from units C and units N, in a molar amount such that the total amount of units C, $N_{philic}$, and F is 100%.

The invention also relates to the use of the random copolymer in a high foaming composition comprising at

least one surfactant, as a foam enhancing agent, preferably as an agent maintaining foam, more preferably as an agent maintaining foam in the presence of soil or external materials, preferably upon soil addition.

The invention also relates to a process of preparing a foamed medium with enhanced foam. The invention also relates to a process of:

- cleaning a textile article by hand, or with a semi automatic equipment,
- cleaning hair and/or skin,
- extinguishing fire,
- tunneling and/or excavating materials, or
- preparing a lightweight material.

The invention provides efficient enhancement of foam, especially maintenance of foam, and more especially upon addition of soil or external materials. As a further advantage the foam enhancement is versatile and can be obtained with a wide range of compositions, as opposed to an effect obtained only on some very particular compositions.

Detailed description of the invention

Definitions

In the present specification, a unit deriving from a monomer is understood as a unit that may be directly obtained from the said monomer by polymerizing. Thus, a unit deriving from an ester of acrylic or methacrylic acid does not encompass a unit of formula -CH-CH(COOH)-, $-CH-C(CH_3)(COOH)-$, -CH-CH(OH)-, $-CH-C(CH_3)(OH)-$, obtained for example by polymerizing an ester of acrylic or methacrylic acid, or a vinyl acetate, and then hydrolyzing. A unit deriving from acrylic acid or methacrylic acid encompasses for example a unit obtained by polymerizing a monomer (for example an alkyl acrylate or methacylate) and then reacting (for example hydrolyzing) to obtain units of formula -CH-CH(COOH)- or $-CH-C(CH_3)(COOH)-$. A unit deriving from vinyl alcohol encompasses for example a unit obtained by polymerizing a monomer (for example a vinyl ester) and then reacting (for example hydrolyzing) to obtain units of formula -CH-CH(OH)- or $-CH-C(CH_3)(OH)-$.

In the present specification, unless provided otherwise, molecular weights of polymers refer to absolute (as opposed to relative determined with reference to polymers of a know molecular weight) weight-average molecular weights determined by gel permeation chromatography with light scattering detection, with an aqueous or organic carrier.

In the present specific, "hydrophobic" for a designated unit or for a designated monomer is understood as its usual meaning: having no affinity for water. More specifically, a hydrophobic monomer or units encompass:

- monomers or units referred to as hydrophobic below, or (if not mentioned)
- monomers or units such that monomer or a homopolymer comprising the same amount of the designated units than the number of the designated units in the copolymer, would form a diphasic macroscopic solution in distilled water at 25°C, at a concentration of 1 % by weight or more.

In the present specific, "hydrophilic" for a designated unit or for a designated monomer is understood as its usual meaning: having affinity for water. More specifically, a hydrophilic monomer or units encompass:

- monomers or units referred to as hydrophilic below, or (if no mentioned)
- monomers or units such the designated monomer or a homopolymer comprising the same amount of the designated units than the number of the designated units in the copolymer, would not form a diphasic macroscopic solution in distilled water at 25°C, at a concentration of 1% by weight or more up to 10%.

The electrical behavior or nature (neutral, anionic or cationic) of units may depend on the pH of the environment of the copolymer, typically the pH of the composition or of a medium where the composition is used. By cationic (respectively anionic) it is meant that the unit is cationic (respectively anionic) whatever the pH, in a range of pH 3-13, preferably pH 1-15. Units comprising a quaternary ammonium group are considered as cationic. By potentially cationic (respectively anionic) it is meant that the unit may be neutral or cationic (respectively anionic) depending on the pH.

Copolymer

The composition of the invention can comprise an amount of the copolymer of the invention of from 0.05 to 5% by weight, preferably of from 0.1 to 2.5%.

The copolymer of the invention is a random copolymer. It is different from a block copolymer. It comprises units

C and units $N_{philic}$. It can comprise some further units F, different from units C and units $N_{philic}$.

Examples of further units that can be comprised are:

- Anionic or potentially anionic units $F_A$, deriving from anionic or potentially anionic monomers $F_A$,
- Different cationic units that comprise a permanent cationic group but do not comprise a quaternary ammonium group, or potentially cationic units, both being referred to as units $F_C$, deriving from different cationic monomers or from potentially cationic monomers, both being referred to as monomers $F_C$,
- Neutral hydrophobic units $F_{Nphobic}$, deriving from neutral hydrophobic monomers $F_{Nphobic}$,
- Zwitterionic units $Z_Z$ comprising both a cationic group and an anionic group, deriving from corresponding monomers $F_Z$.

The copolymer can comprise several different units qualifying as units C (respectively units $N_{philic}$; respectively units F), all of them would then account in the amount of units C (respectively units $N_{philic}$; respectively units F).

If units $F_A$ or units $F_C$ or units $F_{Nphlbic}$ or units $F_Z$ are present, their respective molar amount is preferably of lower than 50%, preferably of lower than 25%. The molar amount of all units F is preferably of lower than 50%, preferably of lower than 25%. In a preferred embodiment units C and units $N_{philic}$ constitute at least 90%, preferably at least 99%, preferably 100% of the units comprised in the copolymer. Thus in a preferred embodiment the copolymer is substantially free of units F.

The monomers that the units derive from are typically ethylenically unsaturated monomers, more preferably from mono-alpha-ethylenically unsaturated monomers or di-allylic cyclopolymerizable monomers.

Units C comprise a quaternary ammonium group. Units C are typically associated to a counter anion, for example a halogen anion such as chloride or bromide, or a sulfate anion, such as methyl sulfate. The quaternary ammonium group has typically formula $-N^+R^1{}_2X^-$- or $-N^+R^1{}_3X^-$, wherein $R^1$, identical or different is $C_1$-$C_4$ alkyl group, optionally substituted (for example by an -OH group), optionally linked directly or via a linking group to a functional group, for example another quaternary ammonium group.

Examples of monomers C, that units C can derive from, include:

- Trimethylammoniumpropylmethacrylate chloride,
- Trimethylammoniumethylacrylamide or methacrylamide chloride or bromide,
- Trimethylammoniumbutylacrylamide or methacrylamide,
- Triméthylammoniumpropylmethacrylamide methylsulfate (MAPTAMeS),
- (3-méthacrylamidopropyl)triméthylammonium chloride (MAPTAC),
- (3-acrylamidopropyl)triméthylammonium chloride (APTAC),
- methacryloyloxyethyl triméthylammonium chloride or methylsulfate,
- acryloyloxyethyl triméthylammonium chloride; or acryloyloxyethyl trimethylammonium (ADAMQUAT Cl ou ADAMQUAT MeS),
- methyldiethylammonium ethylacrylate (ADAEQUAT MeS),
- benzyldimethylammonium ethylacrylate chloride or mehylsulfate (ADAMQUAT BZ 80),
- N,N-dialkyldiallyammonium monomers such as N,N-dimethyldiallylammonium chloride (DADMAC),
- dimethylaminopropylmethacrylamide,N-(3-chloro-2-hydroxypropyl) trimethylammonium chloride (DIQUAT chloride)
- dimethylaminopropylmethacrylamide,N-(3-methylsulfate-2-hydroxypropyl) trimethylammonium (DIQUAT methylsulfate)
- monomer of formula

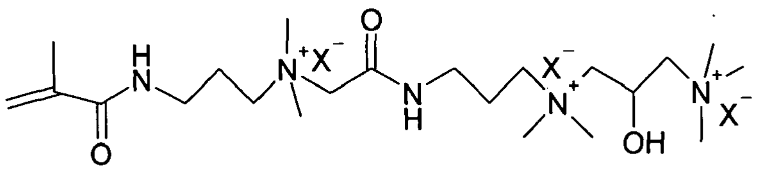

  wherein X- is an anion, preferably chloride or methylsulfate,
- mixtures or associations thereof.

Examples of monomers $N_{philic}$, that units $N_{philic}$ can derive from, include:

- Acrylamide, méthacrylamide,
- vinyl lactame monomers, such a N-vinylpyrrolidone or N-vinylcapromactame,

- vinyl alcohol,
- polyethylene oxide (meth)acrylate (i.e. polyethoxylated (meth)acrylic acid),
- hydroxyalkylesters of alpha-ethylenically-unsaturated, preferably mono-alpha-ethylenically-unsaturated, monocarboxylic acids, such as 2-hydroxyethylacrylate (HEA) or 2-hydroxyethylmethacrylate (HEMA),
- hydroxyalkylamides of alpha-ethylenically-unsaturated, preferably mono-alpha-ethylenically-unsaturated, monocarboxylic acids,
- mixtures or associations thereof.

Examples of potentially cationic monomers $F_C$, that potentially cationic units $F_C$ can derive from, include:

- monomers bearing a tertiary amine group, such as dimethylaminoethyl (meth)acrylate, dimethylaminopropyl (meth) acrylate, ditertiobutylaminoethyl (meth)acrylate, dimethylaminomethyl (meth)acrylamide, dimethylaminopropyl (meth)acrylamide;
- ethylenimine, vinylamine, 2-vinylpyridine, 4- vinylpyridine,
- mixtures and associations thereof.

Examples of neutral hydrophobic monomers $F_{Nphobic}$, that neutral hydrophobic units $F_{Nphobic}$ can derive from, include:

- linear or branched alkyl acrylate or methacrylate, such as methylacrylate, ethylacrylate, n-propylacrylate, n-butylacrylate, methylmethacrylate, ethylmethacrylate, n-propylmethacrylate, n-butylmethacrylate, n-hexylacrylate, n-hexylmethacrylate n-octylacrylate, n-octylmethacrylate, isooctylacrylate, isooctylmethacrylate, 2-ethylhexylacrylate, 2-ethylhexylmethacrylate, and lauryl
- vinyl esters of carboxylic acids, such as vinylacetate, vinyl Versatate®,
- vinylamine amide,
- acrylonitrile,
- vinylaromatic monomers, such as styrene.

The copolymer is preferably soluble in water and/or in the composition. Preferably the amount of units $F_{Nphobic}$, if any, is sufficiently low to allow water solubility and/or solubility in the composition. The copolymer is preferably substantially free of units $F_{Nphobic}$.

Some useful copolymers include copolymers essentially consisting units deriving from

- MAPTAC and Acrylamide (AM),
- DADMAC and Acrylamide (AM),
- DIQUAT Chloride and Acrylamide (AM).

The copolymer can advantageously have a weight-average molecular weight of from 10000 to 2000000 g/mol, preferably of from 100000 to 1000000 g/mol, preferably of from 200000 to 800000 g/mol.

The copolymer can be prepared by any conventional copolymerization process, including a free-radical polymerization process. Such a process typically includes a step of placing unsaturated comonomers in presence of a source of free radicals (initiators for example), preferably with heating and/or controlling the temperature above 25°C, typically in the range 30°C to 90°C. The process can be performed in solution in water. Polymerization can be stopped to control molecular weights by cooling or using agents stopping propagation of free radicals (inhibitors).

The copolymer can be provided in any form, for example it can be provided as an aqueous solution, with a concentration of for example 5-50% by weight.

<u>Surfactant</u>

The composition of the invention can comprise an amount of surfactant of at least 5%, preferably of at least 8%, preferably of at least 10%. The amount of surfactant can be of up the 99%, preferably of up the 60%, by weight.

The surfactant can be an anionic surfactant, an amphoteric surfactant, an amphoteric surfactant (including zwitterionic surfactants), a cationic surfactant or a mixture thereof. The surfactant preferably comprises at least one anionic surfactant.

Surfactants or combination of surfactants suitable for high foaming compositions for the particular intended use are known by the one skilled in the art, and compositions can be designed accordingly. The composition of the invention especially the surfactant(s) therein, can be substantially identical to usual compositions with further addition or use of the copolymer of the invention.

Examples of anionic surfactants that may be mentioned in particular include:

- alkylester sulfonates of formula R-CH($SO_3$M)-COOR' in which R represents a $C_8$-$C_{20}$ and preferably $C_{10}$-$C_{16}$ alkyl radical, R' represents a $C_1$-$C_6$ and preferably $C_1$-$C_3$ alkyl radical and M represents an alkali metal cation (sodium, potassium or lithium), substituted or unsubstituted ammonium (methylammonium, dimethylammonium, trimethylammonium, tetramethylammonium, dimethylpiperidinium, etc.) or an alkanolamine derivative (monoethanolamine, diethanolamine, triethanolamine, etc.). Mention may be made most particularly of methyl ester sulfonates in which the radical R is of $C_{14}$-$C_{16}$;
- $\alpha$-olefin sulfonates containing from 12 to 16 carbon atoms;
- alkyl sulfates of formula $ROSO_3M$, in which R represents a $C_5$-$C_{24}$ and preferably $C_{10}$-$C_{18}$ alkyl or hydroxyalkyl radical, M representing a hydrogen atom or a cation of the same definition as above, and also ethoxylenated (OE) and/or propoxylenated (OP) derivatives thereof, containing on average from 0.5 to 30 and preferably from 0.5 to 10 OE and/or OP units;
- alkylamide sulfates of formula RCONHR'$OSO_3$M, in which R represents a $C_2$-$C_{22}$ and preferably $C_6$-$C_{20}$ alkyl radical, R' represents a $C_2$-$C_3$ alkyl radical, M representing a hydrogen atom or a cation of the same definition as above, and also the ethoxylenated (OE) and/or propoxylenated (OP) derivatives thereof, containing on average from 0.5 to 60 OE and/or OP units;
- saturated or unsaturated $C_8$-$C_{24}$ and preferably $C_{14}$-$C_{20}$ fatty acid salts, $C_9$-$C_{20}$ alkylbenzenesulfonates, primary or secondary $C_8$-$C_{22}$ alkylsulfonates, alkylglycerol sulfonates, the sulfonated polycarboxylic acids described in GB-A-1 082 179, paraffin sulfonates, N-acyl N-alkyltaurates, alkyl phosphates, isethionates, alkylsuccinamates, alkylsulfosuccinates, sulfosuccinate monoesters or diesters, N-acyl sarcosinates, alkylglycoside sulfates or polyethoxycarboxylates,
  the cation being an alkali metal (sodium, potassium or lithium), a substituted or unsubstituted ammonium residue (methylammonium dimethylammonium, trimethylammonium, tetramethylammonium, dimethylpiperidinium, etc.) or an alkanolamine derivative (monoethanolamine, diethanolamine, triethanolamine, etc.);
- alkyl or alkylaryl phosphate esters, for instance Rhodafac RA600, Rhodafac PA15 or Rhodafac PA23 sold by the company Rhodia.

Among the nonionic surfactants that may be mentioned in particular are alkylene oxide condensates, especially of ethylene oxide with alcohols, polyols or alkylphenols; fatty acid esters; fatty acid amides; fatty amines; amine oxides; sugar derivatives such as alkylpolyglycosides or fatty acid esters of sugars, especially sucrose monopalmitate; long-chain tertiary phosphine oxides; dialkyl sulfoxides; sequence copolymers of polyoxyethylene and of polyoxypropylene; polyalkoxylated sorbitan esters; fatty esters of sorbitan, poly(ethylene oxides) and fatty acid amides modified so as to give them a hydrophobic nature (for example fatty acid monoethanolamides and diethanolamides containing from 10 to 18 carbon atoms).

Mention may be made most particularly of

- polyoxyalkylenated (polyethoxyethylenated, polyoxypropylenated or polyoxybutylenated) alkylphenols, the alkyl substituent of which is of $C_{6-12}$ and contains from 5 to 25 oxyalkylene units; examples that may be mentioned include Triton X-45, X-114, X-100 or X-102 sold by Rohm & Hass Co.;
- glucosamides, glucamides and glycerolamides;
- polyoxyalkylenated $C_8$-$C_{22}$ aliphatic alcohols containing from 1 to 25 oxyalkylene (oxyethylene or oxypropylene) units. Examples that may be mentioned include Tergitol 15-S-9 and Tergitol 24-L-6 NMW sold by Union Carbide Corp., Neodol 45-9, Neodol 23-65, Neodol 45-7 and Neodol 45-4 sold by Shell Chemical Co., Rhodasurf IDO60, Rhodasurf LA90 and Rhodasurf IT070 sold by the company Rhodia;
- amine oxides such as $C_{10}$-$C_{18}$ alkyl dimethylamine oxides or $C_8$-$C_{22}$ alkoxy ethyldihydroxyethylamine oxides;
- the alkylpolyglycosides described in US-A-4 565 647;
- optionally polyhydroxylated $C_8$-$C_{20}$ fatty acid amides,
- ethoxylated fatty acids;
- ethoxylated amines.

Examples of cationic surfactants include alkylammonium salts of formula:

$$R^1R^2R^3R^4N^+ X^-$$

in which

- $X^-$ represents a halogen ion, $CH_3SO_4^-$ or $C_2H_5SO_4^-$
- $R^1$ and $R^2$ are identical or different and represent a $C_1$-$C_{20}$ alkyl radical or an aryl or benzyl radical

- $R^3$ and $R^4$ are identical or different and represent a $C_1$-$C_{20}$ alkyl radical, an aryl or benzyl radical or an ethylene oxide and/or propylene oxide condensate $(CH_2CH_2O)_x$-$(CH_2CHCH_3O)y$-H, in which x and y range from 0 to 30 and are never both zero, for instance cetyltrimethylammonium bromide, or Rhodaquat® TFR sold by the company Rhodia.

Examples of zwitterionic surfactants include quaternary aliphatic ammonium derivatives, especially 3-(N,N-dimethyl-N-hexadecylammonio)propane 1-sulfonate and 3-(N,N-dimethyl-N-hexadecylammonio)-2-hydroxypropane 1-sulfonate.

Examples of amphoteric surfactants include betaines, sulfobetaines and fatty acid and imidazole carboxylates and sulfonates.

The following surfactants can be preferred:

- alkyldimethylbetaines, alkylamidopropyldimethylbetaines, alkyldimethylsulfobetaines or alkylamidopropyldimethylsulfobetaines, for instance Mirataine CBS sold by the company Rhodia, and the products of condensation of fatty acids and of protein hydrolyzates;
- alkylamphoacetates or alkylamphodiacetates in which the alkyl group contains from 6 to 20 carbon atoms;
- amphoteric derivatives of alkylpolyamines, for instance Amphionic XL® sold by Rhodia, and Ampholac 7T/X® and Ampholac 7C/X® sold by Berol Nobel.

It is preferred that the composition comprises at least 5% by weight of at least one anionic surfactant, preferably of:

- an optionally ethoxylated linear alkylbenzene sulfonate, or
- an optionally ethoxylated linear alkyl sulfate.

<u>Compositions and other ingredients</u>

The composition can be for example:

- a foaming cosmetic composition to clean skin and/or hair,
- a shaving composition,
- a hand dish-washing composition,
- a composition for cleaning vehicles,
- a composition for laundry by hand,
- a composition for semi-automatic laundry,
- a fire-extinguishing composition,
- a civil engineering composition for tunneling and/or excavating materials, or for preparing a lightweight material.

Such compositions are known by the one skilled in the art.

The composition can comprise further ingredients, aside from the copolymer of the invention and of the surfactants. Further ingredients suitable for high foaming compositions for the particular intended use are known by the one skilled in the art, and compositions can be designed accordingly. The composition can be substantially identical to usual compositions with further addition or use of the copolymer of the invention. Examples of further ingredients include:

- pH controlling agents,
- viscosity modifiers, thickeners,
- caring agents (for textile articles or for skin and/or hair),
- deposition aids for caring agents,
- builders,
- solvents,
- dyes,
- perfumes,
- solid particles,
- optical brighteners,
- soiling suspension agents,
- soil release polymers,
- bleach,
- hydrotropes,
- wetting agents

- enzymes,
- biocides,
- stabilizers,
- preservatives,
- salts, and
- mixtures thereof.

The composition can comprise water and/or at least one water-compatible solvent. The term "water-compatible solvent" means any solvent which, when mixed with water, forms a single transparent phase at room temperature.
Water and/or the solvent may represent up to 99.5% of the total mass of said composition; the minimum amount of water and/or water-compatible solvent is usually 1%. When it is a matter of a water/solvent mixture, said solvent may represent up to 80% of the weight of said mixture. Said solvent can be chosen from $C_2$-$C_8$ aliphatic monoalcohols or polyalcohols, and ethers thereof. Examples of solvents that may especially be mentioned include ethanol, propanol, isopropanol, butanol, 2-butoxyethanol, diethylene glycol, 1-butoxyethanol-2-propanol and diethylene monobutyl ether.
The composition, especially when it is a concentrated liquid composition, may also comprise at least one polymer to control the viscosity and/or stability of the composition and or of the foam, for instance polyacrylic acids or water-soluble salts thereof with a weight-average molecular mass of from 1000 to 5 000 000 g/mol, block copolymers of ethylene oxide-propylene oxide with a weight-average molecular mass of up to 30 000 g/mol, polyethylene glycols with a molecular mass of at least 400 g/mol, and biopolymers with a molecular mass of at least 10 000 g/mol; when it is present, said polymer may represent from 0.01% to 10% of the mass, as dry matter, of the concentrated liquid composition.
The term "concentrated composition" means any composition that may be diluted during use.
As examples of polymers capable of controlling the viscosity and/or stability of foams, mention may be made of

- polyacrylic acids or salts thereof, for instance the Carbopols from B.F. Goodrich, especially Carbopol 941, Carbopol 801, Carbopol 907, Carbopol 910, Carbopol 934 and Carbopol 940;
- block copolymers of ethylene oxide-propylene oxide, for instance the Antarox products from Rhodia, especially Antarox F-88;
- biopolymers, for instance guar gum, gum arabic, xanthan gum, rheozan gum, welan gum, carrageenans, and cellulose or guar derivatives (carboxymethylcellulose, hydroxyethylcellulose, hydroxypropyl guar, carboxymethyl guar or carboxymethylhydroxypropyl guar).

Examples of thickeners include thickeners of gum type (especially a xanthan gum introduced to a concentration of from 0.1 % to 3%). Mention may be made especially of:

- optionally acidic cleaning agents such as mineral acids (phosphoric acid, sulfamic acid, hydrochloric acid, hydrofluoric acid, sulfuric acid, nitric acid or chromic acid) or organic acids (acetic acid, hydroxyacetic acid, adipic acid, citric acid, formic acid, fumaric acid, gluconic acid, glutaric acid, glycolic acid, malic acid, maleic acid, lactic acid, malonic acid, oxalic acid, succinic acid or tartaric acid) and acid salts (sodium bisulfate);
- and also one or more of the following minor ingredients: a preserving agent for preventing the growth of microorganisms in the product, a colorant, a fragrance and/or an abrasive agent.

Compositions for hand dish-washing composition, for laundry by hand, or for semi-automatic laundry, will typically comprise organic or mineral detergence builders, such as sodium carbonate, zeolites, sodium silicates, mineral phosphates (for example sodium tripolyphosphate), polyacrylates or citrates. As regards compositions for washing laundry in a semiautomatic machine, the composition can typically organic or mineral builders (sodium carbonate, zeolites, sodium silicates, mineral phosphates, polyacrylates or citrates), anti-redeposition agents, anti-soiling agents, color-transfer blockers and nonionic softeners, further to the surfactant(s) and the copolymer.
The composition is typically free (less than 1% by weight, preferably less than 0.5%, preferably less than 0.1%, preferably less than 0.01% by weight, preferably less than 0.001% by weight, preferably none) of an anti-foaming agent. Anti foaming agents are known by the one skilled in the art. Examples include polyorganosiloxane-based (silicones) anti-foaming agents.
In a preferred embodiment, the composition has a foaming index of at least 20, preferably of at least 40. The procedure for determining for foaming index of a composition is detailed in the examples.
According to one embodiment, the composition if substantially free (less than 1% by weight, preferably less than 0.5%, preferably less than 0.1%, preferably less than 0.01% by weight, preferably less than 0.001% by weight, preferably none) of a diamine compound.
The composition can be in any form suitable for the final use. The composition can be for example a liquid,

gel, powder, granules, or tablet form. The composition can be in an aqueous or nonaqueous form. Foam is typically formed in the presence of water, by agitation and/or by propelling the composition.

The expression "high foaming composition" is taken in the broad sense herein. It may be, for example:

- a liquid composition comprising water (or a water/water-compatible solvent mixture), forming a foam on the surface to be treated by spraying, using apparatus equipped with a nozzle (spray)
- a liquid composition comprising water (or a water/water-compatible solvent mixture), forming a foam by shear, obtained by manual or vortex mixing
- a solid, gel or aqueous or nonaqueous liquid composition forming a foam after dilution in water or with water and shear (rubbing, manual mechanical, or vortex mixing).

Use

The composition can be used according the typical procedures, known by the one skilled in the art and/or by end users, with using typical means. For example the composition can be used, in the following procedures:

- cleaning skin and/or hair,
- shaving composition, including with dispensing from an aerosol mean,
- dish-washing by hand,
- cleaning textile articles by hand, in the private, industrial or institutional sphere
- cleaning textile articles by hand with using a semi automatic equipment, including according to the procedures describes above, in the private, industrial or institutional sphere,
- cleaning vehicles, including private cars, at home or at a service facility, or trucks,
- fire-extinguishing, including with dispensing from an aerosol mean
- tunneling and/or excavating materials,
- preparing a lightweight material, including preparing a material by foaming a composition comprising setting material (s) (hydraulic binders such as cements, plasters, etc or polymeric cross-linkable materials) and allowing the material (s) to set while the foam in present.

Upon use, one will typically implement a step of foaming the composition. The step of foaming can be performed with nozzle or aerosol means (with a propelling gas). In most cases, one will typically dilute the composition, preferably with water, prior to or during foaming. Foaming can be performed by agitating, applying a shear, and/or vortexing, with manual means, or with motorized equipments.

Dilution can be typically of 10 to 50000 times the volume of the composition, depending on the intended use. The surfactant concentration, after dilution, can typically be of from 0.0001% to 10%, preferably from 0.001% to 5% and most particularly from 0.005% to 2%.

In semi-automatic laundry the dilution rate can be typically of from 10 to 1000, preferably of about 100. In hand dish washing of in laundry by hand the dilution rate can be typically of from 10 to 20000, depending on the user.

Some illustrative but non-limiting examples are provided hereunder for the better understanding of the invention.

## Examples

Foaming Index and Foam maintenance tests

The Foaming index and the foam maintenance of a tested composition are determined with the following cylinder apparatus, according to the following protocol.

The apparatus has six parallel Plexiglas® cylinders fixed to a rotating frame. Each cylinder has an internal diameter of 9cm, with an internal height of 29cm. Each is equipped with a graduated scale allowing a measure of a foam height. The cylinders are fixed to a rotating frame, each cylinder occupying equivalent positions in the frame. The frame is moved by an electric engine, to rotate with cylinders, along an axis that perpendicular to the main axis of the cylinders, cutting the cylinders in the middle of their length, in the plane of the frame. The composition in a cylinder flows within said cylinder and hits its extremities (the bottom and the top) during the rotation of the cylinder, thereby generating turbulence that leads to the creation of foam. Each cylinder is closed with a removable lid, in which a 8mm hole is drilled in order to allow the introduction of additives (such as soil). This hole is closed with a rubber cork when the cylinders are rotated. The foam height is determined by reading the graduated scale when the cylinders are in a vertical position after rotation:

$$\text{Foam height} = \text{height of (foam + liquid composition)} - \text{height of liquid composition.}$$

The Foam Height Unit (FHU) is defined as the following: 10 FHU correspond to a foam height of 25 mm.

The rotation speed is set to 20 rpm (rotations per minute). The cylinders are rotated by series of 10 rotations (which each last 30 seconds) followed by 3 minutes of rest between each series to allow a measure of the foam height (done at the end of the 3 minutes) and optionally a soil addition.

The cylinders contain 500 ml of the composition to be tested. The composition to be tested has a starting temperature controlled at 20°C.

<u>Foaming Index Measurement Protocol:</u>

A cylinder is carefully pored with 500 ml of the composition to be tested, with avoiding formation of foam in the cylinder upon poring. The frame holding the cylinders is then rotated in six series of ten rotations (total of 60 rotations), each series being followed by a waiting time of 3 minutes. The foam height in the cylinder is measured at the end of the 3 minutes.

The Foaming Index is defined as the foam height, given in FHU, after the $6^{th}$ waiting time of 3 minutes.

In order to improve precision, every experiment is repeated twice, the Foaming Index is the average.

<u>Foam maintenance protocol</u>:

After the $6^{th}$ series of (ten rotations + 3 minutes waiting times), one carries out a soil addition implement by adding 5 droplets (0.15g calibrated with a lab balance) of liquid and warm soil (80°C) into the cylinder. The cylinder is then given 10 subsequent rotations at 20 rpm. The foam height is then measured 3 minutes after the 10 rotations (total of 60+10=70 rotations). One can repeat the soil addition implement with adding the soil just after the foam height measure and just before the subsequent rotation.

This rotations/wait/measure/soil addition implement is repeated until the foam height reaches a value of less 10 FHU.

In order to improve precision, every experiment is repeated twice, with the average being reported.

One plots or reports the foam height as a function of the number of rotations (the soil is added only after 60 rotations. The foam high (foam maintenance) upon addition of soil is especially of interest. A slow foam decrease indicates a stabilization of the foam, in presence of soil.

<u>Soil composition</u>

The soil used in the example is a synthetic sebum simulating greasy soils such as soils coming from the human skin, mixed with a clay (bentonite) simulating particular soils (dust...). The weight ratio between the synthetic sebum and the clay is 12/4.

Composition for 950g of synthetic sebum:

| Ingredient | g | % |
|---|---|---|
| Palmitic acid | 100 | 10,5 |
| Stearic acid | 50 | 5,3 |
| Sebacid acid | 50 | 5,3 |
| Oleic acid | 100 | 10,5 |
| Linoleic acid | 50 | 5,3 |
| Paraffin wax (40-50°C) | 100 | 10,5 |
| Coconut oil (copprah) | 100 | 10,5 |
| Squalen | 50 | 5,3 |
| Mineral oil | 200 | 21,1 |
| Soja oil | 50 | 5,3 |
| Colza oil | 100 | 10,5 |

Preparation: the ingredients are placed in a Pyrex® beaker, and heated to 80°C during 15 minutes, with a magnetic stirring on a heating plate. The liquid obtained is transparent and lightly colored. Upon cooling, it forms a white and opaque paste that can be stored for several weeks in a freezer.
For the foam maintenance evaluations, the soil is prepared by melting 12g of synthetic sebum at 80°C with magnetic stirring, and by adding 4g of clay to that transparent mixture. The now opaque and slightly viscous soil is stirred and maintained at 80°C during the entire operation. A fresh sample of this mixture is prepared for every new experiment; the soil is never kept for more than one hour.

Example 1: Foaming index and foam maintenance of a model composition for semi automatic laundry, without candidate polymer

One prepares 1000g of the following composition:

- 0,8g LABS (linear alkyl benzene sulfonate, anionic surfactant)
- 0,5g Rhodasurf L7/90 (nonionic surfactant)
- 3g Sodium TriPolyPhosphate
- 4g $NA_2SO_4$
- tap water: to 1000g.

The foaming index and the foam height are reported in table 1 below.

Table 1

| Number of rotations | Foam height (FHU) | Cumulated Number of additions of soil |
|---|---|---|
| 10 | 56 | 0 |
| 20 | 64 | 0 |
| 30 | 67 | 0 |
| 40 | 70 | 0 |
| 50 | 71 | 0 |
| 60 | 75 = Foaming index | 0 |
| 70 | 46 | - 1 |
| 80 | 21 | 2 |
| 90 | 16 | 3 |
| 100 | 8 | 4 |
| 110 | 6 | 5 |
| 120 | 5 | 6 |
| 130 | 4 | 7 |

The foam height reaches the limit of 10 only after 4 soil additions.

Example 2: Foaming index and foam maintenance of a model composition for semi automatic laundry, with candidate polymer

The candidate polymer is a copolymer of 95 mol% of Acrylamide and 5 mol% of MAPTAC, with a weight average molecular weight of 400 000 g/mol.
One prepares 1000g of the following composition:

- 0,8g LABS (linear alkyl benzene sulfonate, anionic surfactant)
- 0,5g Rhodasurf L7/90 (nonionic surfactant)
- 3g Sodium TriPolyPhosphate
- 0.05g candidate polymer (as active - introduced as pre-dissolved solution in water)
- 4g $NA_2SO4$

- tap water: to 1000g.

The foaming index and the foam height are reported in table 2 below.

Table 2

| Number of rotations | Foam height (FHU) | Cumulated Number of additions of soil |
|---|---|---|
| 10 | 54 | 0 |
| 20 | 61 | 0 |
| 30 | 66 | 0 |
| 40 | 68 | 0 |
| 50 | 69 | 0 |
| 60 | 71 = Foaming index | 0 |
| 70 | 58 | 1 |
| 80 | 46 | 2 |
| 90 | 34 | 3 |
| 100 | 27 | 4 |
| 110 | 19 | 5 |
| 120 | 12 | 6 |
| 130 | 8 | 7 |

The foam height reaches the limit of 10 only after 7 soil additions.

The polymer does not modify significantly the amount of foam obtained in the absence of soil. When the soil is added, the foam level decreases significantly slower when the polymer is present than when it is absent (comparing examples 1 and 2). This means that the foam in the presence of soil is more stable in with the polymer than without polymer.

## Claims

1. Highly foaming composition comprising:

    a) at least one surfactant, and
    b) a random copolymer comprising:

    - from 0,1 to 20% molar, preferably from 1 to 10%, of cationic units C deriving from a cationic monomer C, comprising a quarternary ammonium group,
    - from 80 to 99,9% molar, preferably from 90 to 99%, of neutral hydrophilic units $N_{philic}$ deriving from a neutral hydrophilic monomer $N_{philic}$, and
    - optionally further units F different from units C and units N, in a molar amount such that the total amount of units C, $N_{philic}$, and F is 100%.

2. A composition according to claim 1, wherein units C and units $N_{Philic}$ constitute at least 90%, preferably at least 99%, preferably 100% of the units comprised in the copolymer.

3. A composition according to any of the preceding claims, wherein the monomers are ethylenically unsaturated monomers.

4. A composition according to any of the preceding claims, wherein monomer C is:

    - (3-méthacrylamidopropyl)triméthylammonium chloride,
    - N,N-dimethyldiallylammonium chloride,
    - dimethylaminopropylmethacrylamide,N-(3-chloro-2-hydroxypropyl) trimethylammonium chloride,

- (3-acrylamidopropyl)triméthylammonium chloride,
- acryloyloxyethyl triméthylammonium chloride, or
- a mixture thereof.

**5.** A composition according to any of the preceding claims, wherein monomer $N_{phile}$ is:

- Acrylamide,
- hydroxyethylacrylate, hydroxyethylmethacrylate,
- vinylpyrrolidone, or
- a mixture thereof.

**6.** A composition according to any of the preceding claims, wherein the copolymer is a random copolymer of (3-méthacrylamidopropyl)triméthylammonium chloride and acrylamide.

**7.** A composition according to any of the preceding claims, wherein the copolymer has a weight-average molecular weight of from 10000 to 2000000 g/mol, preferably of from 100000 to 1000000 g/mol, preferably of from 200000 to 800000 g/mol.

**8.** A composition according to any of the preceding claims, having an amount of copolymer of from 0.05 to 5% by weight, preferably of from 0.1 to 2.5%.

**9.** A composition according to any of the preceding claims, having an amount of surfactant of at least 5%, preferably of at least 8%, preferably of at least 10%.

**10.** A composition according to any of the preceding claims, wherein the surfactant comprises at least 5% by weight, with reference to the total weight of the composition od an anionic surfactant, preferably an optionally ethoxylated, linear alkylbenzene sulfonate, or alkyl sulfate.

**11.** A composition according to any of the preceding claims, being substantially free of an anti-foaming agent.

**12.** A composition according to any of the preceding claims, wherein it has a foaming index of at least 20, preferably of at least 40.

**13.** A composition according to any of the preceding claims, being:

- a foaming cosmetic composition to clean skin and/or hair,
- a shaving composition,
- a hand dish-washing composition
- a composition for laundry by hand,
- a composition for semi-automatic laundry,
- a composition for cleaning vehicles,
- a fire-extinguishing composition,
- a civil engineering composition for tunneling and/or excavating materials, or for preparing a lightweight material.

**14.** The use of the copolymer as defined in claims 1 to 7, in a composition as defined in claims 1 or 8 to 13.

**15.** The use according to claim 14, as foam enhancing agent.

**16.** The use according to claim 14 or 15, as an agent maintaining foam in presence of soil, preferably upon soil addition.

**17.** A process preparing a foamed medium comprising the step of diluting with water a composition according any of claims 1 to 13 and foaming.

**18.** A process according to claim 17, wherein the step is carried out in a process of:

- cleaning a textile article by hand, or with a semi automatic equipment,
- shaving,
- cleaning hair and/or skin,

- extinguishing fire,
- tunneling and/or excavating materials,
- preparing a lightweight material,
- cleaning a vehicle, or
- dish-washing by hand.

European Patent Office

# EUROPEAN SEARCH REPORT

Application Number
EP 07 29 1118

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 98/26864 A (INST FRANCAIS DU PETROLE [FR]; ARGILLIER JEAN FRANCOIS [FR]; AUDIBERT) 25 June 1998 (1998-06-25)<br>* page 7, lines 23-25 *<br>* page 8, lines 13-20 *<br>* claim 6; examples 7-9 *<br>* page 1, lines 9-13 *<br>----- | 1-18 | INV.<br>A61K8/04<br>A61K8/81<br>A61Q5/02<br>A61Q19/10 |
| X | WO 96/19966 A (ISP INVESTMENTS INC [US]; CURTIS HELENE IND INC [US])<br>4 July 1996 (1996-07-04)<br>* page 8, lines 6-14 *<br>* page 9, line 11 - page 10, line 12 *<br>* page 15 *<br>* examples *<br>----- | 1-18 | |
| X | DE 100 56 909 A1 (WELLA AG [DE])<br>17 May 2001 (2001-05-17)<br>* examples *<br>----- | 1-18 | |

| TECHNICAL FIELDS SEARCHED (IPC) |
|---|
| A61K<br>A61Q |

The present search report has been drawn up for all claims

| Place of search | Date of completion of the search | Examiner |
|---|---|---|
| Munich | 19 February 2008 | Mitchell, Gemma |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 07 29 1118

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2008

| Patent document cited in search report | | Publication date | Patent family member(s) | | | Publication date |
|---|---|---|---|---|---|---|
| WO 9826864 | A | 25-06-1998 | AU | 7736598 | A | 15-07-1998 |
| | | | EP | 0900124 | A1 | 10-03-1999 |
| | | | FR | 2757426 | A1 | 26-06-1998 |
| | | | US | 6172010 | B1 | 09-01-2001 |
| WO 9619966 | A | 04-07-1996 | AU | 703824 | B2 | 01-04-1999 |
| | | | AU | 4608996 | A | 19-07-1996 |
| | | | CA | 2203401 | A1 | 04-07-1996 |
| | | | CN | 1171044 | A | 21-01-1998 |
| | | | EP | 0805671 | A1 | 12-11-1997 |
| | | | JP | 11500417 | T | 12-01-1999 |
| DE 10056909 | A1 | 17-05-2001 | BR | 0105224 | A | 25-06-2002 |
| | | | EP | 1206929 | A2 | 22-05-2002 |
| | | | JP | 2002154933 | A | 28-05-2002 |
| | | | US | 2002122783 | A1 | 05-09-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 1082179 A
- US 4565647 A